# EUROPEAN PATENT APPLICATION

(11) **EP 4 666 940 A1**
(43) Date of publication of application: **24.12.2025**
(21) Application number: 24212652.2
(22) Date of filing: 13.11.2024
(51) Int. Cl.: A61B 5/06, A61B 5/367, A61B 5/00, A61B 5/287, A61B 5/349

(54) **SYSTEMS OF MAPPING ELECTROPHYSIOLOGICAL LANDMARKS WITHIN A HEART**

(30) Priority: 19.06.2024 US 202418747552
(71) Applicant: Biosense Webster (Israel) Ltd., 2066717 Yokneam (IL)
(72) Inventor: GOVARI, Assaf, 2066717 Yokneam (IL)
(74) Representative: Carpmaels & Ransford LLP

(57) **Abstract**

Systems and methods to locate Electro-Physiological (EP) landmarks in real-time during electro-physiological mapping of a heart are disclosed. The method includes: tracking locations within the heart of a catheter's distal end, recording a reference location of a reference landmark in the heart, and determining a region of interest (ROI) for locating the sought EP landmark relative to the reference landmark. As long as the distal end is within the ROI, an IEGM signal measured by from the distal end and a concurrent ECG signal are processed to determine whether a predetermined characterizing signal feature of the sought EP landmark is manifested within a predetermined time window portion of the IEGM signal relative to a referenced timing provided by the ECG. In case the characterizing signal feature is manifested, the location, from which the IEGM signal was measured, is identified as a location of the sought EP landmark.

## Description

### TECHNOLOGICAL FIELD

The present invention relates generally to electrophysiological (EP) mapping of a heart, and particularly to identification, mapping and visualization of EP landmarks such as the His bundle, its right and left bundles, the Christa Terminalis, as well as other EP landmarks.

### BACKGROUND AND GENERAL DESCRIPTION

It is well known to use ablation catheters to create tissue necrosis in cardiac tissue to correct cardiac arrhythmias (including, but not limited to, atrial fibrillation, atrial flutter, atrial tachycardia and ventricular tachycardia). Arrhythmia can create a variety of dangerous conditions including irregular heart rates, loss of synchronous atrioventricular contractions and stasis of blood flow which can lead to a variety of ailments and even death.

During the cardiac ablation one or more lesions are produced in the certain locations of a patient's heart tissue in order to prevent/suppress stray electrical signals causing arrhythmia. However, the heart includes and electrical conduction system including certain electrophysiological (EP) landmarks which play critical role in proper heart operation, and whose ablation should be avoided. For example, the His bundle, otherwise known as the bundle of His, or His, is a part of the heart muscle that originates near the orifice of the coronary sinus (CS) that serves a role in transmitting electrical impulses from the atrioventricular (AV) node, which located between the atria and the ventricles, to the ventricles of the heart. Other such EP landmarks may include for instance the sinoatrial node (SA) node, the atrioventricular (AV) node and the crista terminalis.

The His bundle for example is positioned at a vulnerable location in the heart, which if ablated by mistake, could result in detrimental and unwanted effects on the electrical conduction system of the heart. During conventional ablation procedures, physicians often manually tag the His bundle as well as possibly other EP landmarks, to identify their locations within the heart so that their ablation can be avoided during the procedure. Such manual tagging is tedious, time consuming and may least to erroneous identification (e.g. due to false positive reads of IEGM signals which may appear to look like signals from the tagged EP landmark).

Conventional automated or partly-automated techniques for real-time identification of EP landmarks during EP mapping are often error prone and may result in false positive identifications. Indeed, none-real-time techniques of EP landmark identification, which are based on post processing of cardiac electrophysiological map (e.g. such as a local activation time (LAT) map and/or bipolar/unipolar potential map) already obtained via EP mapping procedures and/or other diagnostic procedures, may be more reliable. However, use of such techniques for EP landmark identification may be cumbersome and time consuming as they require to firstly conduct an extensive prior cardiac electrophysiological mapping procedure before the EP landmarks can be identified (e.g. to facilitate ablation). A need exists for an automated and reliable system and methods to automatically detect EP landmark(s) reliably and with accuracy in real time during cardiac EP mapping and/or ablation procedures.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** is a schematic, pictorial illustration of a system for electrophysiological (EP) mapping, in accordance with an exemplary embodiment of the present invention;
**FIG. 2A** is a schematic, pictorial illustration of a heart showing several landmarks including examples of EP landmarks which may be mapped by the system of **FIG. 1****,** as well as landmarks which may be used for the EP mapping, in accordance with an exemplary embodiment of the present invention;
**FIG. 2B** is a graphical illustration of concurrent intracardiac electrogram (IEGM) and electrocardiogram (ECG) signals, which are processed during the EP mapping by the system of **FIG. 1****;**
**Fig. 3A** is a block diagram exemplifying in more details a configuration of a processor of the of system of **FIG. 1** which is used for the electrophysiological mapping according to embodiments of the present invention; and
**FIG. 3B** is a flow diagram that schematically illustrating a method for real time mapping of landmarks in accordance with an exemplary embodiment of the present invention.

### DETAILED DESCRIPTION OF EMBODIMENTS

Some electrophysiological (EP) disorders, such as cardiac arrhythmia, may be manifested in episodes of irregular EP signals, such as irregular intracardiac electrograms (IEGMs). A mapping catheter having one or more electrodes at its distal end, may be used for cardiac EP mapping of the heart. The mapping catheter is used to acquire IEGMs and to thereby detect (e.g. and locate) cardiac tissue(s), which cause the irregular IEGMs, (e.g. arrhythmogenic tissue(s)), so that they can be ablated.

The mapping catheter is also used to detect and locate electrophysiological (EP) landmarks of the heart (e.g. being part of the heart's electrical conduction system) whose ablation should typically be avoided in order not to disrupt proper operation of the heart. One such EP landmark is the for example the His bundle, which is a part of the Purkinje fibers located between the heart chambers. Other EP landmarks whose ablation should typically be avoided may include the sinoatrial node (SA) node, the atrioventricular (AV) node and the crista terminalis, left and right bundle branches. Therefore, it is important to locate these EP landmarks and identify them on a map of the heart presented to the physician, prior to the ablation, so he can avoid ablating those tissue regions.

However, some EP landmarks are difficult to identify reliably from IEGMs captured by the mapping catheter (e.g. since their characteristic signal features in the IEGM are small or may resemble IEGM signal features obtained from other tissue regions). For instance, it may be difficult to reliably identify the His bundle since its identifying signal peak in the IEGM signal is weak. As a result, identification of the His bundle primarily based on such a small signal feature (peak) alone is prone to many false positive results (as such small peak may be detected/sensed from other tissue locations as well, and/or due to noise).

Embodiments of the present invention as described in the following, provide systems and method for automatic real-time detection and location of one or more EP landmarks during EP mapping. The technique of the invention utilizes an EP mapping catheter having at least one IEGM electrode at its distal end by which IEGM signals can be measured from the heart tissue near thereat. EP mapping catheter is equipped with a position sensor, and/or is associated with another positioning technology (e.g. ACL described below) by which the location(s) of the IEGM electrode(s) at its distal end can be tracked within the heart during the EP mapping procedure. In order to provide accurate detection and location of EP landmarks in real time, the technique of the invention utilizes both spatial and temporal references for processing the IEGM signals acquired by the catheter in search EP landmarks whose locations are sought. Advantageously by utilizing both the spatial and temporal references, most/all false positive identifications are avoided, thus allowing for real time detection of the desired EP landmarks during the mapping. Even more advantageously, when utilizing the technique of the invention to identify/locate a plurality of the EP landmarks in real-time, the location(s) of any one or more first EP landmark(s) identified during the mapping, may be incorporated-in/added-to the spatial references used of the EP landmark identification and/or features of their IEGM signals may be used as temporal reference, thus further assisting in the efficient detection of location of other(s) of the sought EP landmarks. Implementations in which the technique of the invention is performed in real time during the EP mapping, may therefore facilitate shortened duration of the EP mapping procedure (e.g. as compared to techniques where EP landmarks are search for only after the EP mapping is complete, i.e. compared to non-real-time techniques), since it allows the EP mapping to be conducted only until all the required EP landmarks and possibly other disordered tissue locations were identified (e.g. while not necessarily requiring to yield a full electrophysiological map of a tissue region of interest, as may be required in conventional techniques that rely on post-processing of cardiac EP maps for the identification of the EP landmarks).

To this end, as indicated above the technique of the invention relies on both spatial and temporal references to provide real-time detection of the sought EP landmarks. As for the spatial reference, each EP landmark is generally known to be located at a certain specific region of the heart, which may be within certain distance ranges from one or more other heart landmarks (anatomical or electrophysiological landmarks). During the EP mapping, the catheter's location is tracked, and landmarks via which it passes are identified (either automatically or via input from the physician). For instance, during the mapping the physician may indicate when the catheter passes certain anatomical landmarks such as the inferior vena cava (IVC) or the entry into the atrium, or other landmarks, and the locations of such landmarks may be registered as spatial references. Alternatively, or additionally, reference anatomical landmarks may be identified automatically for instance using real time imaging (e.g. ultrasound) during the mapping, and the locations thereof may be included as reference landmarks. Moreover, the locations of certain EP landmarks already located by the technique of the invention may also be registered as spatial references to be used for identifying and location further EP landmarks during the mapping.

Additionally, in some embodiments also timing of characteristic IEGM signal features of certain already identified EP landmarks, may be registered as temporal references to be used in further identification of additional EP landmarks during the mapping procedure (e.g. by /comparing the timing IEGM signal features suspected to be sourced by such an additional sought EP landmark, with the timing of a characteristic IEGM signal feature of an already identified EP landmarks to determine whether the suspected IEGM signal feature of the additional landmark falls within an acceptable time interval relative to the timing of the characterizing feature of the already identified EP landmark (note that in such embodiments the acceptable time intervals between characterizing IEGM signal features of various EP landmarks may be provided in reference data used by the system).

In this regard, it should be noted that the phrase anatomical landmarks is used herein to designate anatomical structures having recognizable shapes/forms which can be identified, by the physician or by the system, during the mapping (e.g. based on imaging or on the catheter movement/manipulation performed when passing near thereat). The phrase EP landmarks is used herein to designate parts of the heart electrical conduction system, which do not necessarily have distinctive/recognizable shape/form and whose locations can be determined from distinctive electrophysiological signal features appearing in IEGM measurements obtained from their vicinities.

The technique utilizes spatial reference data indicative of spatial relations (e.g. relative positions/distance-ranges between each EP landmark whose location is to be sought by the system, and one or more reference landmarks. Accordingly, with the accumulation of one or more reference landmarks whose locations within the heart are identified during the EP mapping, regions of interest (ROI) at which the sought EP landmarks are expected to reside, may be defined, based on the locations of the reference landmark and the spatial relations between them and the sought EP landmarks as indicated in the spatial reference data.

During the EP mapping, the location of the distal end of the catheter (i.e. of IEGM electrode(s) thereon) is tracked to determine whether it is within the ROI at which one or more of the sought EP landmarks is expected to reside. For instance, the distance/relative-position of the catheter's IEGM electrode(s) relative to certain reference landmark(s) via which it had traversed are may be monitored/processed, to determine (i.e. based on the spatial relations indicated above) when it is in the ROI. Then, IEGM signals obtained by the catheter from the ROI are processed utilizing a temporal reference ECG signal such to identify whether the IEGM signals obtained from the ROI manifest the characteristic signal features of the sought EP landmark with proper timing relative to the temporal reference. Thus, many false positive identifications of the sought EP landmark are avoided by utilizing the spatial references to restrict the search for the temporal characterizing signal features of the sought EP landmarks only to IEGM signals from within the respective ROI of the sought EP landmarks, which is determined based on the reference landmarks and their spatial relations to the sought EP landmark (e.g. as IEGM signals which may be resemble the characteristic signal of the sought EP landmark but originate from other regions are a-priori overruled from being originated from the sought EP landmark).

Accordingly, IEGM signals measured by the electrode(s) at the distal end of the catheter from location(s) in the ROI at which the sought EP landmark might reside (i.e. the ROI satisfying the reference spatial relations relative to the identified reference landmark(s)), are processed to determine whether they manifest signal features (signal peaks and/or troughs) matching the characterizing signal feature(s) expected to originate from the sought EP landmark. Predetermined data indicative of the characterizing signal feature(s) of the sought EP landmark (also referred to herein interchangeably as reference signal feature(s)) may include for example properties such as time intervals ΔTᵢ (e.g. relative to an ECG reference timing or relative to timings of signal features of other EP landmarks) and optionally also the shapes (e.g. heights/widths/spectral-content) of certain signal features expected to appear in an IEGM signal measured from the sought EP landmark. To this end, the timings of signal features manifested in the IEGM signal may be inferred based/relative-to the timing of certain features (e.g. P and/or QRST complexes) in an ECG signal that serves as a base line temporal reference, and compared with the time interval(s) ΔTᵢ of the corresponding characterizing signal features of the sought EP landmark, to determine their match. Optionally also the shapes (e.g. heights and/or widths) of the manifested signal features are considered to determine their match with the reference signal features.

Thus, according to the technique of the invention, the location of the sought EP landmark is determined/identified when: (i) an IEGM signal measured by the mapping catheter from a location within the ROI at which the sought EP landmark is expected to reside (i.e. from location satisfies the reference spatial relations with other reference landmarks); and (ii) when it manifests signal feature(s) whose timing(s) and optionally also shape(s) match the characteristic signal feature(s) that are expected from the sought EP landmark.

The technique of the invention facilitates real time identification and marking/tagging of EP landmarks during EP mapping procedure, e.g., before the EP map is fully constructed. This enables the EP mapping procedure to be performed interactively, for instance by presenting the already mapped EP landmarks to the physician on the display in real time during the mapping, and optionally also providing real time guidance for directing the catheter's distal end towards the ROI(s) at which respective EP landmarks should be sought. The technique of the invention thereby facilitates an efficient, short and accurate EP mapping procedure (e.g. as the physician is may not need to continue mapping regions of already identified EP landmarks, and may also be guided to search the desired EP landmarks at specific ROI(s)). Moreover, based on the tracking of the catheter's location and the use of the spatial as well as temporal references to accurately identify the sought EP landmarks, the technique reduces/eliminates false positive identifications, while also obviates a need for identification of these EP landmarks by post processing of cardiac electrophysiological maps.

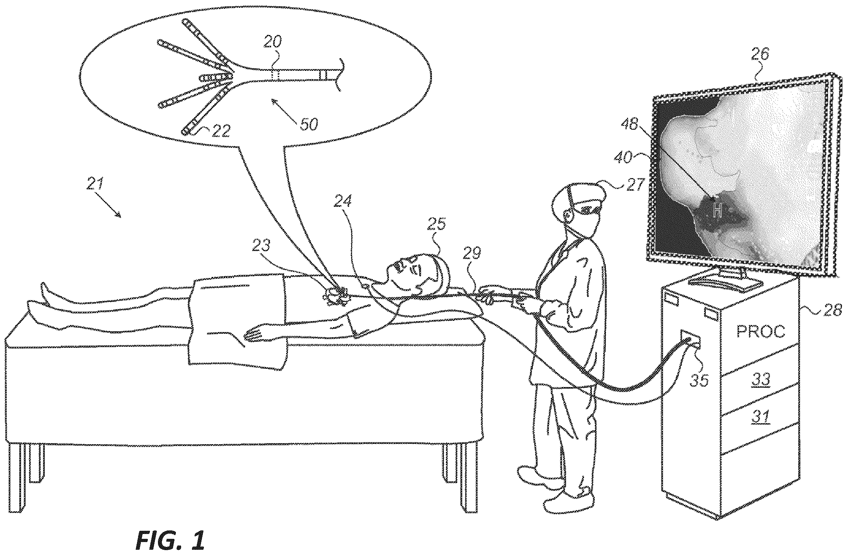

**FIG. 1** is a schematic illustration of a system **21** for electrophysiological (EP) mapping, in accordance with an exemplary embodiment of the present invention. **FIG. 1** depicts a physician **27** using catheter **29** connected to the system **21,** to perform an EP mapping of a cardiac chamber (e.g., a left atrium (LA) and/or a right atrium (RA) and/or a left ventricle (LV), and/or a right ventricle (RV)) of a heart **23** of a patient **25.**

Catheter **29,** which is also referred to herein as an *EP mapping catheter,* may be a therapeutic catheter that has EP mapping capabilities and/or a designated EP mapping catheter. Catheter **29** generally includes at least one mapping/IEGM electrode **22** at its distal end **50,** which is adapted to acquire signals from the tissue of heart **23** in its vicinity. In this none-limiting example catheter **29** includes an electrode array **50** including one or more arms/splines **20,** with mapping/IEGM-electrodes **22** disposed along each of the arms **20.** It should be understood that in various embodiments, different/other types of mapping catheters may be used (e.g. with or without splines and/or having a different number of mapping electrodes **22;** i.e. at least one).

System **21** is also connectable to, or includes, one or more ECG electrodes **24,** typically external/body-surface ECG electrodes, which are adapted to couple to the body surface/skin of patient **25,** and configured to acquire electrocardiograms (ECG) of the patient. For instance, three body-surface ECG electrodes **24** may be coupled to the patient's chest, and another three body-surface ECG electrodes may be coupled to the patient's back (in this none-limiting example, for clarity only one body-surface ECG electrode **24** is illustrated in the figure).

System **21** includes a processor **28** adapted to determine the locations of one or more sought EP landmarks, in real time, during the EP mapping procedure. In the none-limiting example of **Fig. 1****,** the processor **28** for example includes a data acquisition utility **33** (e.g. a signal processor) and an EP landmark Locator processing utility **31.** The data acquisition utility **33** is configured and operable to receive and pre-process signals from the mapping electrode(s), the ECG electrode(s) **24,** and signals indicative of the position of the catheter **29,** and may be adapted to perform various signal processing operations to the signals it receives, such as A/D conversion and/or filtering, and provide data indicative thereof for further processing by the EP landmark locator processing utility **31.** The EP landmark Locator processing utility **31** is adapted to process data indicative of the signals obtained/preprocessed by the data acquisition utility **33** (or data indicative thereof) and based on spatial and temporal reference data, determine the locations of the one or more EP landmarks sought. The EP landmark Locator processing utility **31** may be implemented for example with a general-purpose computerized system **31** including data-storage/memory capable of storing the reference data and a processing unit adapted to execute computer readable code for processing the data/signals obtained from the data acquisition utility **33** to determine the EP landmarks' location(s). Processor **28** may be programmed with software (e.g. downloadable over a network and/or stored on non-transitory tangible media) to carry out the functions described herein.

It should be understood that the configuration of the processor **28** exemplified herein is provided only for clarity and a person of ordinary skill in the art will radially appreciate that the technique of the present invention as described below may also be implemented by processor **28** implementing with other processing configurations.

During the EP mapping procedure, the location of the distal end **50** of the catheter **29,** and more specifically of the mapping electrode(s) thereof, is tracked by processor **28.** The tracking may be performed by means of magnetic positioning technology and/or by means of the Advanced Current Location (ACL) tracking, as described in the following. For instance, in embodiments where magnetic positioning technology is employed to track the distal end **50** of the catheter **29,** the catheter **29** may include a magnetic position sensor **20** embedded in or near its distal end. The position sensor **20** may including three magnetic coils for sensing three-dimensional (3D) position and orientation of the catheter's **29** distal end **50** relative to external magnetic field provided by a location pad (not specifically illustrated in the figure). The location pad may be part of the system **21** and may include magnetic field generator(s) (e.g. coils) generating magnetic fields in a predefined working volume surrounding the patient **25,** based on which the three-dimensional (3D) position of the catheter's **29** distal end **50** may be sensed by the magnetic position sensor **20** of the catheter. Accordingly, real time position of distal end **50** of the catheter **29** may be tracked based on the magnetic fields sensed by the position sensor **20.** Details of the magnetic based position sensing technology are described for example in U.S. Patent Nos. 5,5391,199; 5,443,489; 5,558,091; 6,172,499; 6,239,724; 6,332,089; 6,484,118; 6,618,612; 6,690,963; 6,788,967; 6,892,091, whose disclosures are incorporated herein by reference. Alternatively, or additionally, the location(s) of IEGM mapping electrode(s) **22** inside the heart **23** of the patient may be tracked by processor **28** by utilizing Advanced Current Location (ACL) tracking technology. According to this technology, processor **28** measures the electrical impedances between each mapping-electrode **22** which is to be tracked and external electrodes, such as the ECG electrodes **24,** and finds location coordinates of the tracked mapping mapping-electrode **22** using these impedances. The Advanced Current Location (ACL) tracking technology is described in more details for example in U.S. Pat. No. 8,456,182, whose disclosure is incorporated herein by reference. An example of a system capable of using the sensed impedances between mapping-electrodes **22** body surface ECG electrodes **24** and track their locations utilizing the ACL tracking technology, and/or capable of using magnetic positioning technology to track the position and orientation of the distal end of the catheter, is the CARTO^{®} 3 system (produced by Biosense Webster Inc., Irvine, Calif.). Alternatively, or additionally, system **21** may for example incorporate/combine both tracking methods in order to track the positioning of the individual mapping electrodes with accuracy (e.g. the magnetic position technology may be used to accurately determine the position of the catheter's **29** distal end, and the ACL technology may facilitate with accurate determination of the relative positions of the one or more mapping electrodes **22** relative to the catheter's **29** distal end, thus together providing the accurate position(s) of the mapping electrode(s) **22** within the heart **23.**

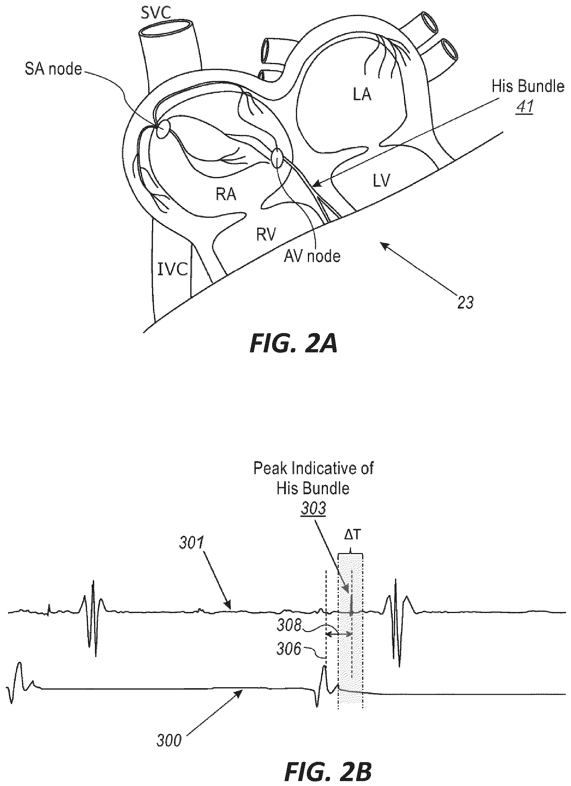

**Fig. 2A** is a schematic illustration of a part of the heart **23** with certain landmarks thereof which may be sought/traversed-by-the-catheter during an EP mapping procedure. The figure illustrates partly the right and left atriums of the heart, **RA** and **LA,** right and left ventricles, **RV** and **LV,** as well as several prominent heart landmarks including in this none-limiting example the superior vena cava **SVC,** inferior vena cava **IVC,** sinoatrial node **SA,** atrioventricular node **AV** and the His bundle **41.** Some of the shown landmarks, such as typically the His bundle **41,** may be the EP landmarks that are sought during typical EP mapping procedure, while others of those landmarks may serve as reference landmark and their locations may be used by the system **21** as spatial references to assist in accurate real time identification of the sought EP landmarks during the EP mapping procedure. During the EP mapping procedure, physician **27** inserts the catheter to the desired chamber of the heart (e.g. atrium/ventricle), typically by following a predetermined path through the heart **23.** For instance, catheter **29** may be inserted via the inferior vena cava **IVC** into the heart **23** and may be progressed along a path in the right atrium **RA,** optionally in case needed mapping EP landmarks at the right atrium, such as the sinoatrial node **SA** and/or atrioventricular node **AV,** and typically further follows through the valve to the right ventricle **RV** to map EP landmarks such as the His bundle and the crista terminalis thereat.

With reference back to **Fig. 1****,** the location of the distal end of the catheter **29,** and more specifically location(s) of one or more of the mapping electrode(s) **22** thereof, is/are tracked (e.g. by means of the magnetic positioning and/or the ACL technologies described above). Additionally, locations of landmarks, which are identified (e.g. anatomical landmarks determined automatically based on imaging/ultrasound cand/or based on input data from the physician **27,** and/or EP landmarks previously identified by the technique of the invention) along the path the distal end **50** of the catheter **29** traverses, may be tagged/recorded as reference landmarks.

The reference landmarks are processed by the system **21** based on predetermined spatial reference data in order to determine respective regions of interest (ROI(s)) in which the system **21** should seeks for the sought EP landmark(s). More specifically, the spatial reference data used by processor **28,** includes predetermined data indicative spatial relations (relative distances/locations) between one or more of the reference landmarks and the sought EP landmarks. Accordingly, processor **28** utilizes these reference spatial relations to determine, based on the locations of reference landmarks, which were already located by the system or identified by the physician, to define the ROI(s), at which each sought EP landmark is expected to be located. The spatial relations in the reference data may include for example, for each EP landmark sought, ranges of distances or relative locations at which the sought EP landmark is expected to reside relative to one or more of the reference landmarks. For instance a relative locations provided in polar coordinates of for the location of the sought EP landmark relative to one of the reference landmarks, may be provided in terms of a relative polar vector (Δr, Δθ, Δφ) defining an individual reference ROI in the form of a spherical sector at which the sought EP landmark is expected to reside relative to the reference landmark (as would be appreciated the relative vector locations may be provided in other coordinates, such as cartesian, and/or may be scalars defining ranges of distances Δr, and/or an angular ranges, e.g. Δθ and/or Δφ). With the accumulation of information about locations of additional reference landmarks, for which relative locations are provided in the reference data for each sought EP landmark, the processor **28** may further narrow the actual ROI at which to seek the sought EP landmark by taking the overlap/cross-section of individual reference ROIs associated with each located reference landmark relative to the sought EP. Accordingly the ROI at which to seek the sought EP landmark is estimated with improved accuracy as additional reference landmarks are identified/located during the procedure.

To this end, during the EP mapping procedure, physician **27** typically poses the catheter **29** at one or more locations to allow the electrode(s) **22** at the distal end **50** to acquire IEGM signals, such as atrial electrograms, from the heart tissue at their vicinity. The signals captured by mapping-electrode(s) **22,** as well as signals indicative of the location of the electrode(s) **22** at the distal end **50** (e.g. signals from the magnetic position sensor **20** at the distal end **50,** and or ACL signals) are conveyed to the processor **28.** Based on these signals the processor **28** may link/associate each respective acquired IEGM signal with the heart location from which the signal was acquired. Processor **28** utilizes the reference landmarks (e.g. EP or anatomical landmarks that were already tagged/identified along the path of the catheter **29)** to determine in real time whether the heart location, from which the respective IEGM signal was acquired, is within the ROI at which any one of the sought EP landmarks may reside. In some embodiments the ROI is determined as the region located within predetermined distance range(s) relative to one or more of spatial reference landmarks already identified in the procedure (the distance range(s) being part of the spatial reference data linking the expected location of the sought EP landmark to one or more reference landmarks). In other embodiments the spatial reference data also includes data indicative of the relative position (e.g. distance range as well as directional/angular range) between the sought EP landmark and the one or more reference landmarks, and the ROI of the sought EP landmark may be determined accordingly based the relative positions.

Accordingly, in case the processor **28** determines that an acquired IEGM signal originates from within a ROI associated with at least one of the sought EP landmarks (at which the sought EP landmark might reside), it proceeds to further apply temporal processing to the acquired IEGM signal to determine whether the acquired IEGM signal has signal features with proper timings, characteristic to the sought EP landmark.

In the following description, an example of the temporal processing is described/exemplified to be performed by system **21** by utilizing predetermined temporal reference data indicative of time intervals and optionally also shapes or other identifiers (e.g. frequency-contents/amplitude-level) of characteristic signal features expected to be manifested in IEGM signals from the sought EP landmark. None-the-less, as would be appreciated by those versed in the art, similar temporal processing can also be implemented in system **21** by a suitably trained machine learning processor/module.

**Fig. 2B** shows graphs of an intracardiac electrogram (IEGM) signal **301** and ECG signal **300** used in the temporal processing. The IEGM signal **301** was obtained during an EP mapping procedure from the ROI of the sought EP landmark. In this example the sought EP landmark is the His bundle **41.** The ROI of the His bundle was determined to be at a predefined distance range from the location at which the catheter **29** had entered the atrium (e.g. typically the IVC). The IEGM signal **301** exemplifies an IEGM signal measured by mapping electrodes **22** of the catheter **29** from within the ROI thereof. The electrocardiogram (ECG) signal **300** is concurrently acquired by one or more of the ECG electrodes **24** of the system **21.** The ECG signal **300** provides temporal base line and is used to determine a reference timing **306,** relative to which the timing of signal features in the acquired IEGM signal(s) can be assessed. For instance, timing of a P and/or QRST signal complexes of the ECG signal, and/or of part thereof, as the timing of the R peak in this example, may be used as the reference timing **306.** It should be noted that the ECG feature providing the reference timing **306,** generally repeats in each heart cycle and may thus may be used temporal base line relative to which the timing of signal features in IEGM signals obtained in different heat cycles, and possibly from different heart locations/EP-landmarks, can be compared. Once the location of the catheter's distal end **50** is within the ROI of the His bundle **41,** the processor **28** processes the acquired IEGM signal(s) **301** (or at least the relevant temporal portions thereof) to determine whether the acquired IEGM **301** manifests characteristic signal features of the sought EP landmark (His bundle). In this processing the processor **28** utilizes an ECG signal **300,** that is concurrently obtained by the ECG electrode(s) **24** to determine the reference timing(s) **306** (e.g. for the heart cycle manifested in the signals **300** & **301)** relative to which timings of signal features manifested in the concurrent IEGM signals within the heart cycle are measured. To achieve that and facilitate use of the ECG signal **301** as temporal reference, a synchronization processing may optionally first be performed to temporally synchronize between the **ECG** signal **300** and the concurrent IEGM signal **301** (i.e. set/determine temporal alignment between them and possibly compensate for any time delays/mismatches in the propagation of those signals from the electrodes **22/24** sensing them. The reference timing **306** is determined by identification of predetermined feature (in this example the R peak) in the ECG signal **300.** Although in this specific none-limiting example, the reference timing **306** is based on the timing of the R peak in the ECG, it should be understood that in general other suitable ECG features, such as the P and/or Q of the PQRST ECG complex, may be used as timing reference. Then temporal reference data may be used to determine the time window **ΔT,** relative to the reference timing **306,** at which at least one characterizing signal feature of the sought EP landmark should appear in the IEGM signal **301.**

In this regard it should be noted that the time interval(s) ΔTᵢ of a characteristic signal feature of the sought EP landmark provided in the temporal reference data, may optionally include a plurality of time interval(s) ΔTᵢ relating the expected timing of the characteristic signal feature of the sought EP landmark relative to the reference timing **306** in the ECG signal and/or relative to characteristic signal features of other EP landmarks. The time interval(s) ΔTᵢ relative to the reference ECG timing **306** and the time interval(s) ΔTᵢ relative to other already identified EP landmarks, may be intersected to determine the time window **ΔT** at which the characteristic signal features of the sought EP landmark is expected to appear. More specifically for example, a section/segment of the IEGM signal **301** that corresponds to a reference time interval ΔTᵢ that relates the expected timing of the characteristic signal feature relative to the reference ECG timing **306** may be determined directly relative to the reference timing **306** in the ECG signal. A section/segment of the IEGM signal **301** that corresponds to a reference time interval ΔTᵢ that relates the expected timing of the characteristic signal feature relative to a characteristic signal feature of another, already identified, EP landmarks, may be determined by summing the reference time interval ΔTᵢ with the timing of the characterizing signal feature of the already identified another EP landmarks relative to the reference ECG timing at the heart cycle at which it was identified, thus determining the corresponding section/segment in the current IEGM signal **301.** Accordingly, the sections/segments as may be determined based on the reference data, and optionally also based on the timings of already identified landmarks, may be intersected/overlapped to determine the time window **ΔT** at which the characteristic signal feature of the currently sought EP landmark should be looked for.

The IEGM signal **301** is processed (e.g. utilizing signal feature identification techniques) in order to identify whether the characteristic signal feature of the currently sought EP landmark appears in the designated time window **ΔT** as determined above. In case it those, the system **21** may determine that the IEGM signal **301** indeed originates from the sought EP landmark, and mark/register the location from which it was acquired, as the location of the sought EP landmark. In some implementations reduction in processing load is achieved by processing only relevant portion/segment of the IEGM signal(s) within the designated time window **ΔT.**

It should be noted that although in the above description, the temporal processing performed by system **21** is described as utilizing explicit processing (e.g. signal processing) of the IEGM and ECG signals based on temporal reference data, the invention is not limited to this type of processing, and may alternatively or additionally use trained machine learning module/processor for processing of the IEGM and ECG signal, **301** and **300,** to determine whether the former manifests signal features expected from the sought EP landmark with proper timing relative to the timing set by the later. Accordingly, it should be understood that references made herein to temporal processing of signals based on temporal reference data, may generally refer to signal processing using explicit temporal reference data, and/or to processing using trained machine learning module in which the such/similar temporal reference data was implicitly embedded during prior training. More specifically, a machine learning module, which is trained (e.g. by supervised training) based on suitable training data, may implicitly incorporate therein (e.g. learn) the characteristics of the temporal reference data described above and perform the above identification of whether an IEGM signal manifests the characteristic signal features of a sought EP landmark, even without explicit use of temporal reference data as described above (i.e. as the same may be "learned" thereby during the training). For example, a suitable training data for such machine learning module may include pairs of concurrent ECG signals and IEGM signals originating from samples of the sought EP landmark and from other location, as well as indication of whether the respective IEGM signals originate from the sought EP landmark (whereby the later may be used for supervising the training). A person of ordinary skill in the art, after knowing the present invention, will readily appreciate various techniques by which such machine learning module can be trained to identify whether an IEGM signal originates from a certain sought EP landmark, based on the IEGM signal and/or relevant portion thereof as well as a concurrent ECG signal provided to the machine learning module as input.

In this example the characterizing signal feature of the His bundle **41** (being the sought EP landmark in this case) in the temporal reference data, is signal feature **303,** which is a small narrow peak **303** that is expected to be located within the predetermined time window **ΔT** after the reference timing **306.** In another example the reference data may indicate that activity in this time window **ΔT** above a defined amplitude/intensity threshold or having a predefined frequency content or shape may be sufficient for identification of the sought EP landmark/HIS **41.** In this example data indicative of both the shape of the peak **303** (e.g. its characteristic width and/or amplitude and/or spectral properties) as well as the predetermined time interval ΔTᵢ at which it should be located relative to the reference timing (the R peak in the ECG in this example) were included as part of the temporal reference data that was used to determine whether and identified peak in an IEGM signal obtained from the ROI, is indeed a peak indicative of the His **41.**

Thus, upon determining that the catheter's distal end **50,** is spatially located in a ROI at which one of the sought EP landmarks may reside, the processor **28** processes the IEGM signal(s) **301** acquired therefrom by the electrode(s) **22,** to identify whether it contains signal features that match the characteristic signal feature(s) of the sought EP landmark and whose timing is within the reference time window **ΔT** relative to the ECG's reference timing **306.** The processor **28** may process only the relevant portion(s) of the IEGM signal(s) **301,** which are within time window **ΔT,** thus reducing processing load/complexity and facilitating agile real time identification of the sought EP landmark. In case the acquired IEGM signal(s) contains such features within the reference time window **ΔT,** the processor **28** determines that the IEGM signal is obtained from the sought EP landmark and may mark/tag or otherwise register the location from which the IEGM signal was acquired (e.g. the location of the catheter's distal end, or of specific electrode **22** thereof), as the location of the sought EP landmark.

As indicated above, once certain EP landmark, such as His bundle **41,** was already identified, and in case the system **21** continues to search for additional EP landmarks, it may utilize the identified timing of the characterizing signal feature(s) of the already identified landmarks, as additional temporal references, by which it can narrow down the search for the signal features of the additional landmarks. For instance, timing(s) of the characterizing feature(s) of an already identified landmark (e.g. timing of peak **303** of the found His bundle **41** relative to the ECG signal **300** - see **Fig. 2B****),** may also be used as temporal reference for identification of additional/other sought EP landmark(s). More specifically for instance, once the His **41** is identified, the time difference **308** between its characterizing His feature **303** and the ECG reference timing **306** can be recorded, and further used to infer the timing of the characterizing His feature **303** in any further heart cycle, even when the catheter is not measuring the IEGM from the His (in such further heart cycle the inferred timing would be the ECG reference timing **306** as obtained for the further heart cycle plus the time difference **308** between it and the characterizing His feature **303** as recorder when the His was identified). Accordingly, in general, the timings of signal features of already identified EP landmarks may further be utilized in the technique of the present invention as temporal references assisting the efficient and accurate identification of additional EP landmarks sought. Provided that the temporal reference data used by the system **21** is indicative of reference time interval ΔTᵢ of the expected relative timing between characterizing signal feature of a first EP landmark to a second one, this can be utilized, together with the measured timing of the characterizing signal feature of the first EP landmark, to determine the time window **ΔT** at which to search of the characterizing features of the second EP landmark with improved accuracy - i.e. searching in a narrower time window **ΔT.** Accordingly, with the accumulation of identified EP landmarks during the EP mapping procedure, the data (e.g. **308)** acquired about the timings of their characterizing signal features (e.g. relative to the reference ECG timing **306)** can be used by the system **21** determine narrower time window **ΔT** at which to search for signal features of other sought EP landmarks. Moreover, this also provides personalized fit of the EP mapping procedure to the specific patient being examined since in this case the time window **ΔT** at which a characterizing signal feature of a further sought EP landmark would be searched for, is specifically set relative to signal properties measured from the electrical heart conduction system of the specific patient.

With reference back to **Fig. 1****,** once identifying the sought EP landmark and determining its location, a tag **48** or other indicia may be added by the processor **28,** in real time during the mapping procedure, to a map **40** of the heart **23** that is presented to the physician **27** on display **26.** Accordingly, the physician **27,** being informed that the location of the sought EP landmark was found, may stop searching for that EP landmark and possible proceed with the EP mapping to search for other EP landmarks and/or map the EP properties of other heart tissues of interest. To this end, as indicated above, once the sought EP landmark is located, its location may be added to the list of reference landmark whose locations are known by the system, and accordingly may be further used as spatial reference in case the system **21**/physician **27** proceeds to search and located additional EP landmark(s).

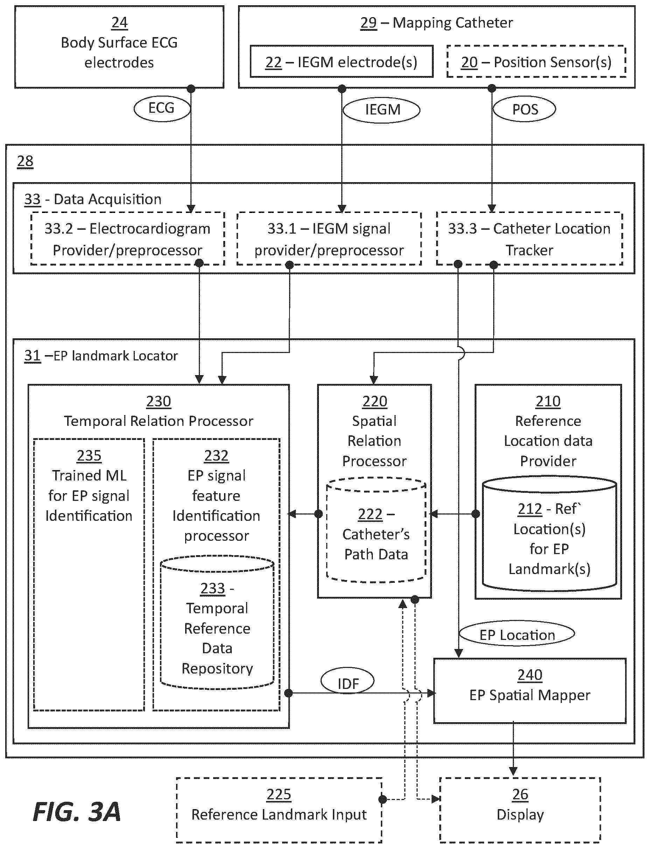

**Fig. 3A** is a block diagram exemplifying a configuration of the processor **28** of the system **21** in more detail. Processor **28** includes a data acquisition utility **33** and an electro-physiological landmark locator utility **31.**

In some example embodiments the data acquisition utility **33** includes an IEGM signal provider/preprocessor **33.1,** an electrocardiogram provider/preprocessor **33.2,** and a catheter location tracker **33.3.**

In this example the IEGM signal provider/preprocessor **33.1** is connectable to one or more mapping electrodes **22** on catheter **29** and adapted to obtain, and optionally preprocess as needed (e.g. digitize/filter), **IEGM** signals measured from the location of the distal end **50** of the catheter **29** within the patient's heart **23.** The electrocardiogram provider/preprocessor **33.2** is connectable to one or more ECG electrodes **24** and adapted to obtain and optionally preprocess as needed (e.g. digitize/filter), **ECG** signals measured from the patient's body. The catheter location tracker **33.3** is adapted to obtain signals **POS** indicative of the location of the catheter's distal end **50,** and more specifically of certain mapping electrode(s) thereof, and process the **POS** signals to determine/assess the electrode(s)' **22** location(s). In some implementations the catheter **29** includes a magnetic positioning sensor **20** optionally furnished at its distal end **50,** and the catheter location tracker **33.3** is connectable to the sensor **20** and adapted to process signals **POS** obtained therefrom to determine the location of the catheter's distal end **50** according to magnetic positioning technology discussed above. Alternatively or additionally, the some implementations the catheter location tracker **33.3** is connectable to one or more of the mapping electrode(s) **22** of the catheter as well as to one or more of the body surface ECG electrodes **24** and/or to other body surface electrode(s), and adapted to obtain signals POS indicative of the impedances between the mapping electrode(s) **22** and the body surface electrode(s) (e.g. **24)** and determine the location of the catheter's distal end **50,** or of the mapping electrode(s) thereat, based on the ACL technology discussed above. Yet alternatively or additionally, in some embodiments the catheter location tracker **33.3** is adapted to implement both the magnetic positioning technology and the ACL technology in combination and thereby determine the position of the catheter's distal end **50,** and/or of the mapping electrode(s) thereat, with improved accuracy. To this end, the catheter location tracker **33.3** operates during the EP mapping procedure **to** track locations within the heart **23** via which a distal end **50** of the catheter is traversed during the mapping.

The electro-physiological landmark locator utility **31** is adapted according to some example embodiments of the present invention, to implement the method **100** as described in more details below in order to locate the positions of one or more sought EP landmarks in real time during the EP mapping. In a none-limiting example embodiment as illustrated in the figure, the electro-physiological landmark locator utility **31** includes a reference-location data provider **210,** spatial relation processor **220,** a temporal relation processor **230** and an EP Spatial Mapper **240.**

The reference-location data provider **210** is typically associated with a data-storage/memory **212** storing spatial reference data indicative of spatial relations (e.g. relative distances/locations between each of the EP landmarks to be sought by the system **21,** and one or more reference landmarks (which may be anatomical landmarks or EP landmarks. To this end, for each EP landmarks to be automatically located by the system, the spatial reference data includes data indicative of its spatial relation relative to at least one other landmark serving as spatial reference for locating the sought landmark. The spatial relation may be a distance range (i.e. scalars) and/or a range of relative locations (i.e. vectors) indicative of a region, at which the sought EP landmark should be located relative to the location of the reference landmark.

The spatial relation processor **220** is connectable/connected to the catheter location tracker **33.3** and adapted to obtain therefrom data indicative of the location(s) within the heart of the mapping electrode(s) **22** at the catheter's distal end **50** from the catheter location tracker **33.3,** and optionally store the path of the distal end **50** and/or of the electrode(s) **22** of the catheter within the heart in a data storage **222.** The spatial relation processor **220** is connectable/connected to the reference-location data provider **210** and adapted to utilize spatial reference data obtained from the reference-location data provider **210** to determine, based on the spatial relations in the spatial reference data, whether the electrode(s) **22** at the catheter's distal end **50** is/are located within a ROI in which one of the sought EP landmarks might reside. In some embodiments the spatial relation processor **220** is associated with a reference landmark input **225** by which it may receive data indicative of the locations of certain reference landmarks within heart **23.** The reference landmark input **225** may for example be connected to a user interface (not specifically shown), and the spatial relation processor **220** may be adapted to receive thereby input from physician **27** about the location(s) of certain reference landmarks. For instance, during the mapping procedure, where physician manipulates the catheter's distal end to enter and path via different regions of the heart, he may recognize certain landmarks (e.g. anatomical) via which it passes (for instance the IVC) and provide input data indicating that the current location of the catheter's distal end is presently near that reference landmark. Alternatively, or additionally, the reference landmark input **225** may for example be connected to an imaging utility, such as an ultrasound imaging utility that is operable to image and possibly identify/recognize based on the images (e.g. using image processing/pattern recognition, or manual input) the locations of certain reference landmarks within the heart, and provide data indicative of the same to the spatial relation processor **220** via the reference landmark input **225.**

In turn, the spatial relation processor **220** may store the locations of reference landmarks received via input **225** in memory **222** (e.g. thus optionally forming record of the path of the catheter, or reference landmarks nearby it had passed, in the memory). Accordingly, the spatial relation processor **220** further utilizes one or more of these locations of reference landmarks stored in memory **222** and the spatial relations (spatial reference data) obtained from the reference-location data provider **210,** to determine whether the catheter's distal end **50** is located at a region (ROI) at which one or more of the sought landmarks might reside. In case it is, the temporal relation processor **230** may operate (as described in more detail below) to process the **IEGM** signals obtained from the catheter's electrode(s) **22** to determine whether the catheter's distal end **50** is located near the sought EP landmark. Optionally, in some embodiments, the spatial relation processor **220** may also utilize the locations of the reference landmarks and the spatial reference data to determine a region in the general area of the distal end **50** of the catheter, at which one or more of the sought EP landmarks might reside, and provide guidance to the physician to move the catheter's distal end **50** towards this region in order to help him in locating the sought EP landmarks. In such embodiments the spatial relation processor **220** may also be connected to display **26** and/or to other output user interface utility (e.g. audio) and may be adapted to thereby provide the physician with guiding indicia to direct him, in real time, to move the distal end of the catheter towards those region(s) at which the sought EP landmarks may be found.

The temporal relation processor **230** is connectable to both the IEGM and the ECG signal providers/preprocessors **33.1** and **33.2** and adapted to receive therefrom concurrent data about the **IEGM** and **ECG** signals measured by the electrodes **22** and **24** respectively. Upon determining, by the Spatial relation processor **220,** that the catheter's distal end **50** is located at a heart region of interest (ROI), at which one of the sought EP landmarks might reside, temporal relation processor **230** operates to process the **IEGM** and **ECG** signals obtained from the ROI to determine whether the **IEGM** signal manifests signal features indicative of it being sensed from the sought **EP** landmark. For that purpose, the **ECG** signal may serve to determine reference timing relative to which the timing of signal features can be assessed.

In certain embodiments, temporal relation processor **230** optionally includes an EP signal features identification utility **232** that is associated with a temporal reference data repository **233** storing temporal reference data. The temporal reference data is indicative of reference signal feature(s) expected to be manifested in **IEGM** signals originating from each sought EM landmarks and the reference time interval(s) ΔTᵢ(s) at which the reference signal feature(s) are expected. The reference time interval(s) may be designated relative to certain reference timing(s) to be determined from the **ECG** signal (e.g. a timing of a certain signal feature of the ECG signal) and/or optionally relative to the timing of characterizing signal features of other sought EP landmarks). In such embodiments, upon obtaining indication from the spatial relation processor **220** that a certain obtained **IEGM** signal is acquired from a ROI at which one of the sought EP landmarks might reside, the EP signal features identification utility **232** operates to process that **IEGM** signal utilizing the temporal reference data and a concurrently obtained **ECG** signal to determine whether the **IEGM** signal manifests the characteristic signal features (peaks/troughs) associated with the sought EP landmark with timing relative to the reference timing, matching/within the reference time interval(s) **ΔT** indicated for those reference features in the temporal reference data (as illustrated for instance in **Fig. 2B****).**

As indicated above, in some implementations the reference signal features also include data indicative of the shape(s) of the characteristic signal feature(s) of the sought EP landmark, and the EP signal features identification utility **232** operates to determines their existence in the IEGM signal based on their shape in addition to their timing.

To this end, for each of the EP landmarks to be sought by the system **21,** the temporal reference data repository **233** may store temporal reference data including signal features such as peaks and/or troughs that are expected to appear in an IEGM signal measured from the ROI of the sought EP landmark, as well as data indicative of an expected time window **ΔT** at which those peaks and/or troughs are expected to appear relative to the ECG's reference timing. Additionally, in some implementations the temporal reference data may also include data indicative of the shape (e.g. spectral content, characteristic width and/or characteristic amplitude) of some of the characteristic signal features. The EP signal features identification utility **232** may implement a signal feature detection algorithm (e.g. peak/troughs detection algorithm) that can be applied to the acquired IEGM signals to detect/identify signal features (peaks/troughs) of predetermined characteristics (e.g. amplitude/width/shape) therein, and determine their timing. Any suitable peak/troughs detection algorithm may be used for this purpose, including for example known in the art amplitude-based and gradient-based algorithms, as well as other algorithms such as machine learning/pattern-recognition algorithms trained for signal feature detection/identification. Peak/troughs detection may be carried out, for example, using smoothing and then fitting a known function (e.g., a polynomial) to the waveform. Alternatively or additionally, processor **28** can match a known feature shape to the waveform. Further alternatively or additionally, peaks and/or troughs can be detected by finding zero-crossings (i.e., local maxima) in the differences (slope sign change) between a point and its neighbors.

For instance, with reference the characterizing peak **303** of the His bundle shown in **Fig. 2B****,** as this peak **303** is a relatively narrow, processor **28** (EP signal features identification utility **232)** can distinguish the peak by passing IEGM signal **301** through a high pass filter to extract only narrow sharp peaks, which could be indicative of the His bundle peak, from the IEGM signal. Then processor **28** (EP signal features identification utility **232)** determines if the timing of any of such extracted peaks relative to the ECG's reference timing **306** falls within the reference time window **ΔT** at which the His peak is expected. To this end, the reference timing **306** may be determined by EP signal features identification utility **232** (by processor **28)** by employing similar feature detection algorithm as described above on the reference ECG signal **300** to identify, and determine the timing of, a certain predetermine signal complex thereof. For instance, the timing of any of the so-called P and/or R peaks and/or the QRST complex in the ECG signal, may serve as the reference timing **306.** EP signal features identification utility **232** then computes the time difference **308** between the identified feature/peak **303** in the IEGM signal **301** and the reference timing **306** of the reference ECG signal **300,** and in case it falls within the predetermined reference time interval **ΔT** at which a corresponding reference feature is expected in an IEGM signal originating from the sought EP landmark, the EP signal features identification utility **232** may determine that the IEGM signal originates from the sought EP landmark (provided that the IEGM signal also originates from a tissue location that satisfies the above indicated spatial conditions), and output indication **IDF** (signal/data) indicative of the same. In the example of **Fig., 2B****,** as shown in the figure, the peak **303** matches the characterizing feature of the His bundle, and indeed also falls within the time window **ΔT** at which it is expected.

Alternatively, or additionally, in certain embodiments, temporal relation processor **230** may optionally include a trained machine learning utility **235** trained to receive the **IEGM** and **ECG** signals as inputs and identify whether the **IEGM** manifests signal features matching those expected from IEGM signals originating from the sought EP landmark with proper timing relative to the timing of the ECG signal. For that the trained machine learning utility **235** may for instance implement a neural network suitably trained to determine based on the input **IEGM** and **ECG** signals, whether the **IEGM** signal originates from the sought EP landmark. As will readily be appreciated by those versed in the art of machine learning and/or pattern recognition after knowing the present invention, such as trained machine learning utility **235** may be implemented by supervised training of one or more neural networks based on training data that includes pluralities of concurrent **IEGM** originating from the sought EP landmarks as well as from other heart regions along with concurrent acquired **ECG** signals, and supervision data indicative of whether the training **IEGM** signals originate from the sought landmarks. By training, the neural networks of the machine learning utility **235** actually learns to identify whether the **IEGM** signal provided thereto as input manifests signal features matching those expected from the sought EP landmark with proper timing relative to the **ECG** signal provided as input, and yields output data indicative of the same. To this end, the temporal relation processor **230** processes the **IEGM** and **ECG** signals measured by the electrodes **22** and **24** respectively and thereby determines whether the **IEGM** signal originates from the sought EP landmark, and outputs indication **IDF** (signal/data) indicative of the same.

EP Spatial Mapper **240** is configured/operable to obtain the indication(s) **IDF** about identified EP landmark(s) from the temporal relation processor **230** in real time, and is also adapted to receive to receive the data indicative of the location of the catheter's distal end **50** from which the respective **IEGM** signal was acquired, from the catheter location tracker **33.3.** Upon receiving indication **IDF** about the **IEGM** signal origination from a certain sought EP landmark, EP Spatial Mapper **240** obtains the location of the catheter from which the **IEGM** signal was acquired, and accordingly marks/sets an indicia/tag **48** indicative of that sought EP landmark in a proper location on a map of the heart **23,** or of part thereof, which is displayed, or to be displayed, on display **26.** For example, the EP Spatial Mapper **240** may tag the found EP landmark with a tag **48** (e.g. "H" exemplified in **Fig. 1** to designate the His) on the heart/EP map **40** shown on the display and/or color the region of the map **40** with color coding indicative of the type of identified landmark. Accordingly, system **21** provides physician **27** with real time indication about EP landmarks identified along the path of the catheter within heart **27** during the EP mapping procedure.

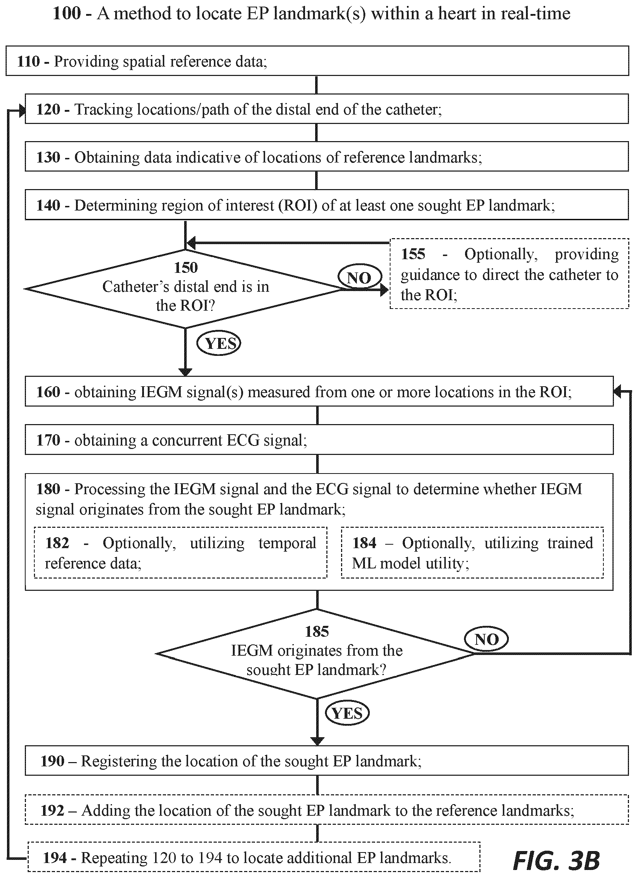

**Fig. 3B****,** is a flow diagram illustrating in self-explanatory manner a method **100** to locate Electro-Physiological landmark(s) within a heart in real-time, according to an embodiment of the present invention. The method **100** may be implemented by the processor **28** of system **21** described above. In operation **110** spatial reference data indicative of spatial relations between one or more reference heart locations one or more sought EP landmarks to be located during the mapping procedure are provided. As indicated in operation **120,** the location of the catheter's distal end **50** is tracked during the EP mapping procedure.

Operation **130** is carried out during the EP mapping procedure, to obtain/receive data indicative of locations of reference landmarks (electro-physiological and/or anatomical) in the patient's heart. The locations of some reference landmark may be identified for example based on input from the physician, and/or by an additional system, such as an imaging utility, and/or may include the locations EP landmarks already identified by method **100** along the path of the catheter **29.** Alternatively, or additionally, the locations of certain landmarks, such as the entry to the atrium from the superior vena cava (SVC) and/or the inferior vena cava (IVC), may be assessed by the system **21,** by monitoring IEGM signals acquired by the catheter while it passes from a certain blood vessel (e.g. SVC/IVC) to the heart chamber. Considering that IEGM signals are generally not acquired from within blood vessels, such as the SVC/IVC, and given predetermined data about the blood vessel (e.g. IVC) via which the catheter is inserted in to the heart in the mapping procedure, the system **21** can monitor the sensed IEGM signals, and upon detecting that IEGM signals are sensed by the catheter, determine that the catheter's distal end **50** is located just at the entry from that blood vessel heart into the heart chamber/atrium it connects to, and accordingly may automatically identify this location as a spatial reference location.

Thus, in operation **130,** which may be carried out throughout the mapping procedure, data about reference landmark locations is accumulated based on one or more information sources, for instance based on input from the physician and/or from an imaging utility, and/or based on IEGM monitoring assisted by predetermined/input data about the path/vessel(s) via which the catheter is inserted to the heart.

Based on the identified reference landmark locations, operation **140** is carried out to determine, utilizing the spatial reference data, region(s) of interest **ROI**(s) at which at least one of the EP landmarks sought in the EP mapping procedure should reside. Operation **150** is carried out during the procedure to determine, based on the location of the distal end **50,** which is tracked (see **120** above), whether the distal end is within a **ROI** of a sought EP landmark determined in **140.** In case it is not, optional operation **155** may be conducted to provide physician **27** with guidance (such as guiding indicia; e.g. arrow on the display **26)** to guide him to towards the ROI, and operation 150 repeats. Otherwise, in case the catheter's distal end is within the ROI at which one of the sought EP landmarks should reside, the method proceeds to operation **160** to **190,** to locate the sought EP landmark in the ROI based on signal features of one or more IEGM signals obtained from one or more locations therein. In operation **160** at least one IEGM signal measured by the catheter **29** from one or more successive locations in the ROI is obtained. Additionally in operation **170** an ECG signal measured substantially concurrently from the patient's body/skin is also obtained. The obtained IEGM and ECG signals are then processed in operation **180** to determine whether the IEGM signal originates from the sought EP landmark. This processing accounts for the temporal relation(s) between signal features of the IEGM signal relative to the ECG signal (e.g. relative to certain features thereof such as the Q and/or PQRST complexes) and/or relative to the timing of certain signal feature(s) in IEGM signal(s) of other sought EP landmarks in IEGM signals which were already identified by the system. The temporal processing determines whether the IEGM signal has features whose timing, relative to a reference timing expressed by the ECG signal or by IEGM signals of already identified EP landmarks, match the expected timing of those signal features for a IEGM signal obtained from the sought EP landmark. The temporal relations, may include one, or more, time intervals ΔTᵢ(s) relating the timing of a signal feature in the IEGM of the sought EP landmark to the timings of one or more signal features in the EGC signal and/or in the IEGM signals of other EP landmarks. Given the ECG signal as well as optionally the IEGM signals of already identified EP landmarks, the processor **28** computes one or more of these time intervals ΔTᵢ(s), and intersects them to yield the time window ΔT designating the portion of the IEGM signal at which the characterizing signal feature of the sought EP landmark should be sought. Accordingly, the processor **28** then processes the IEGM signal (or at least the portion/segment thereof within the time window **ΔT** of the IEGM signal) in search for signal feature therein which match the characterizing signal feature of the sought EP landmark. To this end, as indicated above, processing load associated determining a match between features in the IEGM signal and the characterizing signal feature of the sought EP landmark is typically alleviated, by processing only the relevant portion/section of the IEGM signal which is within the time window **ΔT.**

As indicated in the optional operations **182** and **184** respectively, the operation **180** may be expressly performed utilizing predetermine temporal reference data indicative of expected time intervals ΔTᵢ(s) at which characterizing signal features of the sought EP landmark should occur relative to signal feature(s) in the ECG or in IEGMs from other EP landmarks; and/or utilizing a neural network/machine-learning model trained to recognize the timing of those features. Optionally also the shape(s) of the signal features in the IEGM signal are accounted for to determine their match to expected signal features from the IEGM signal, as described above. As indicated in **185** operations **160** to **180** may be repeated until a match is determined **in 180.** Then once the IEGM signal is determined to originate from the sought EP landmark, in operation **190,** the heart location from which it was acquired by the catheter (as can be determined based on the tracking **120),** is identified/set as the location of the sought EP landmark, and an indication (e.g. an indica/tag) marking that may be presented on a heart map on the display **26.** In case additional EP landmarks should be sought in the EP mapping procedure, the method may proceed to optional operations **192** and **194** in which the location of the EP landmark may be added to the list of reference landmarks (as obtained in 130), and the method may be continued for locating the additional sought EP landmarks by repeating the operations **120** to **190.**

Although exemplary embodiments of the present invention are described above, for the sake of concreteness and clarity, with specific reference to the elements of system **21,** the principles of the present invention may similarly be applied in other EP mapping systems with suitable sensing capabilities. All such alternative embodiments are considered to be within the scope of the present invention.

### Examples

Example 1. A method to locate Electro-Physiological (EP) landmark within a heart in real-time during an electro-physiological mapping of a patient's heart utilizing a catheter, the method includes:
**(a)** tracking locations within the heart via which a distal end of the catheter is traversed during said electro-physiological mapping;
**(b)** recording reference location of at least a first spatial reference landmark in the heart;
**(c)** determining a region of interest (ROI) for locating the sought EP landmark based on a pre-defined distance range relative to the at least first spatial reference landmark;
**(d)** as long as at least one electrode at the distal end is within the ROI:
   **i)** obtaining data indicative of an IEGM measured by the at least one electrode in synchronization with data indicative of an ECG signal;
   **ii)** identifying reference timing based on the ECG signal;
   **iii)** identifying a portion of the IEGM signal being within a predetermined time window relative to the referenced timing; and
   **iv)** determining whether a predetermined characterizing signal feature of the sought EP landmark is manifested in the portion of the IEGM;
   **v)** upon determining that said characterizing signal feature is manifested within the time window, identifying a location of the at least one electrode as a location of the sought EP landmark; and
(e) marking said location of the sought EP landmark on a map of the heart rendered on a display.

Example 2. The method according to Example 1 in which the reference timing is identified as a timing of a predetermine feature in a PQRST complex of the ECG signal.

Example 3. The method according to Example 1 or 2, further including determining the predetermined time window based on predefined temporal reference data indicative of at least one time interval at which the characterizing signal feature should appear in an IEGM signal obtained from the sought EP landmark.

Example 4. The method according to Example 3 in which the predefined temporal reference data further includes data indicative of at least one of a shape, frequency content and amplitude of the characterizing signal feature. Accordingly, the determining of whether the characterizing signal feature is manifested within the time window includes processing the portion of the IEGM signal to determine whether it comprises a signal feature having at least one of a shape, a frequency content and amplitude, matching the temporal reference data.

Example 5. The method according to any one of Examples 1 to 4 including a use of machine learning (ML) processor/model that is trained to determine whether the IEGM signal originates from the sought EP landmark.

Example 6. The method according to Example 5 in which the ML processor is adapted to carry out at least part of operation (d) (which is indicated in Example 1).

Example 7. The method according to any one of Examples 1 to 6 further includes recording at least one additional reference location of at least one additional spatial reference landmark in the heart. The method includes determining the ROI based on pre-defined distance ranges relative to the at least first reference location and the at least one additional reference location.

Example 8. The method according to any one of Examples 1 to 7 further includes locating at least one additional sought EP landmark, by further carrying out operation (d) as long as the at least one electrode is within a second region of interest (ROI) determined based on at least one second pre-defined distance range relative to at least one of said reference location and another reference location.

Example 9. The method according to Example 8 in which the location of the sought EP landmark serves as said another reference location relative to which the second ROI for locating the at least one additional sought EP landmark is determined.

Example 10. The method according to Example 8 or 9, in which carrying out of the operation (d) for locating the additional sought EP landmark, further includes determining a second predetermined time window at which a second characterizing signal feature of the additional sought EP landmark should appear in a second IEGM signal obtained therefrom. The second predetermined time window is determined based on predefined temporal reference data indicative of one or more time intervals at which the second characterizing signal feature should appear in the second IEGM signal, by intersection of the one or more time intervals.

Example 11. The method according to Example 10, in which the one or more time intervals include at least one of: a time interval relative to the reference timing, and time interval relative to a timing of the characterizing signal feature of a sought EP landmark that has already been identified.

Example 12. The method according to any one of Examples 1 to 11 in which the sought EP landmark is at least one of the following: His bundle, right bundle, left bundle, Christa Terminalis, SA node, and AV node.

Example 13. The method according to any one of Examples 1 to 12 being adapted to operate in real-time during an intracardiac electrogram mapping procedure having a predetermined mapping path traversing near one or more of the at least first reference spatial landmark within the heart.

Example 14. The method according to Example 13 wherein the predetermined mapping path follows from the inferior vena cava (IVC) into the right atrium to identify the His bundle, and further follows to identify the right and left bundles and to identify the Christa Terminalis.

Example 15. The method according to any one of Examples 1 to 14 wherein the EP mapping is conducted utilizing a catheter having one or more electrodes capable of measuring the IEGM signals at a distal end thereof.

Example 16. The method according to any one of Examples 1 to 15 further including providing guidance indicia for directing the catheter towards the ROI of the sought EP landmark, upon determining that the at least one electrode is not within ROI of the sought EP landmark.

Example 17. A system to locate Electro-Physiological (EP) landmark within a heart in real-time during an electro-physiological mapping of a patient's heart utilizing a catheter. The system includes one or more processors that are configured and operable to carry out the following:
**(a)** track locations within the heart via which a distal end of the catheter is traversed during the electro-physiological mapping;
**(b)** record a reference location of at least a first spatial reference landmark in the heart;
**(c)** determine a region of interest (ROI) for locating the sought EP landmark based on a pre-defined distance range, relative to the at least first spatial reference landmark;
**(d)** as long as at least one electrode at said distal end is within said ROI:
   **i)** obtain data indicative of an IEGM measured by the at least one electrode in synchronization with data indicative of an ECG signal;
   **ii)** identify reference timing based on the ECG signal;
   **iii)** identify a portion of the IEGM signal that is within a predetermined time window relative to the referenced timing; and
   **iv)** determine whether a predetermined characterizing signal feature of the sought EP landmark is manifested within the portion of the IEGM signal;
   **v)** upon determining that the characterizing signal feature is manifested, identify a location of the at least one electrode as a location of the sought EP landmark; and
**(e)** mark the location of the sought EP landmark on a map of the heart rendered on a display.

Example 18. The system according to Example 17 in which the one or more processors are adapted to carry out at least one of the following in order to determine whether the IEGM signal originates from the sought EP landmark:
- determine the predetermined time window based on predefined temporal reference data indicative of the characterizing signal feature with at least one time interval at which it should appear in an IEGM signal obtained from the sought EP landmark; and process the portion of the IEGM signal to determine, based on manifestation of the predetermined characterizing signal feature therein, whether the IEGM signal originates from the sought EP landmark; and
- utilize trained machine learning (ML) model to determine whether the IEGM signal originates from the sought EP landmark.

Example 19. The system according to Example 17 or 18 in which the one or more processors are adapted to locate at least one additional sought EP landmark by carrying out the following:
- determine at least one second region of interest (ROI) based on one or more second pre-defined distance ranges at which the additional sought EP landmark is expected to reside relative to at least one of: the reference location and another reference location; and
- carry out operation (d) (similar to that indicated in Example 17) for locating the additional sought EP landmark as long as the at least one electrode is within said second ROI.

Example 20. The system according to Example 19 wherein at least one of the following:
- The location of the sought EP landmark serves as said another reference location relative to which the second ROI for locating the at least one additional sought EP landmark, is determined.
- Upon carrying out of the operation (d) for locating the additional sought EP landmark, the one or more processors determine a second predetermined time window at which a second characterizing signal feature of the additional sought EP landmark should appear in a second IEGM signal obtained therefrom. The second predetermined time window is determined based on one or more predefined time intervals indicative of expected timing of the second characterizing signal feature relative to at least one of: the reference timing and a timing of a characterizing signal feature of a sought EP landmark that has already been identified.

Example 21. The system according to any one of Examples 17 to 20 adapted to carry out the method according to any one of Example 1 to 16.

It will be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A system to locate Electro-Physiological (EP) landmark within a heart in real-time during an electro-physiological mapping of a patient's heart utilizing a catheter, the system comprises one or more processors that are configured and operable to carry out the following:
**(a)** track locations within the heart via which a distal end of said catheter is traversed during the electro-physiological mapping;
**(b)** record a reference location of at least a first spatial reference landmark in the heart;
**(c)** determine a region of interest (ROI) for locating said sought EP landmark based on a pre-defined distance range, relative to the at least first spatial reference landmark;
**(d)** as long as at least one electrode at said distal end is within said ROI:
**i)** obtain data indicative of an IEGM measured by the at least one electrode in synchronization with data indicative of an ECG signal;
**ii)** identify reference timing based on the ECG signal;
**iii)** identify a portion of the IEGM signal that is within a predetermined time window relative to the referenced timing; and
**iv)** determine whether a predetermined characterizing signal feature of the sought EP landmark is manifested within the portion of the IEGM signal;
**v)** upon determining that said characterizing signal feature is manifested, identify a location of the at least one electrode as a location of the sought EP landmark; and
**(e)** mark said location of the sought EP landmark on a map of the heart rendered on a display.

2. The system according to claim 1 wherein said one or more processors are adapted to carry out at least one of the following to determine whether said IEGM signal originates from the sought EP landmark:
- determine said predetermined time window based on predefined temporal reference data indicative of said characterizing signal feature with at least one time interval at which it should appear in an IEGM signal obtained from the sought EP landmark; and process the portion of the IEGM signal to determine, based on manifestation of the said predetermined characterizing signal feature therein, whether said IEGM signal originates from the sought EP landmark; and
- utilize trained machine learning (ML) model to determine whether said IEGM signal originates from the sought EP landmark.

3. The system according to claim 1 or claim 2 wherein said one or more processors are adapted to locate at least one additional sought EP landmark; whereby for locating said at least one additional sought EP landmark the one or more processors carry out the following:
- determine at least one second region of interest (ROI) based on one or more second pre-defined distance ranges at which said additional sought EP landmark is expected to reside relative to at least one of said reference location and another reference location; and
- carry out operation (d) for locating said additional sought EP landmark as long as the at least one electrode is within said second ROI.

4. The system according to claim 3 wherein at least one of the following:
- said location of the sought EP landmark serves as said another reference location relative to which said second ROI for locating said at least one additional sought EP landmark, is determined.
- upon carrying out of said operation (d) for locating the additional sought EP landmark, the one or more processors determine a second predetermined time window at which a second characterizing signal feature of the additional sought EP landmark should appear in a second IEGM signal obtained therefrom; wherein said second predetermined time window is determined based on one or more predefined time intervals indicative of expected timing of said second characterizing signal feature relative to at least one of said reference timing and a timing of the characterizing signal feature of the sought EP landmark as was already been identified.

5. The system according to claim 1 wherein said reference timing is identified as a timing of a predetermine feature in a PQRST complex of said ECG signal.

6. The system according to claim 1 or claim 5 wherein the one or more processors are configured and operable to determine said predetermined time window based on predefined temporal reference data indicative of at least one time interval at which said characterizing signal feature should appear in an IEGM signal obtained from the sought EP landmark, optionally:
wherein said predefined temporal reference data further comprises data indicative of at least one of a shape, frequency content and amplitude of said characterizing signal feature, and wherein the one or more processors are configured and operable to determine whether the characterizing signal feature is manifested within the time window by processing said portion of the IEGM signal to determine whether it includes a signal feature having at least one of a shape, a frequency content and amplitude matching the temporal reference data.

7. The system according to any one of claims 1, 5 and 6 wherein the one or more processors are configured and operable to utilize machine learning (ML) processor trained to determine whether said IEGM signal originates from the sought EP landmark,
optionally wherein the ML processor is adapted to carry out at least part of operation (d).

8. The system according to any one of claims 1 and 5 to 7 wherein the one or more processors are configured and operable to record at least one additional reference location of at least one additional spatial reference landmark in the heart; and determine said ROI based on pre-defined distance ranges relative to said at least first reference location and said at least one additional reference location.

9. The system according to any one of claims 1 and 5 to 8 wherein the one or more processors are configured and operable to locate at least one additional sought EP landmark, by further carrying out operation (d) as long as the at least one electrode is within a second region of interest (ROI) determined based on at least one second pre-defined distance range relative to at least one of said reference location and another reference location.

10. The system according to claim 9 wherein said location of the sought EP landmark serves as said another reference location relative to which said second ROI for locating said at least one additional sought EP landmark, is determined.

11. The system according to claim 9 or claim 10, wherein the one or more processors are configured and operable to carry out operation (d) for locating the additional sought EP landmark by determining a second predetermined time window at which a second characterizing signal feature of the additional sought EP landmark should appear in a second IEGM signal obtained therefrom; wherein said second predetermined time window is determined based on predefined temporal reference data indicative of one or more time intervals at which said second characterizing signal feature should appear in the second IEGM signal, by intersection of said one or more time intervals,
optionally wherein said one or more time intervals include at least one of: a time interval relative to said reference timing, and a time interval relative to a timing of a characterizing signal feature of a sought EP landmark that has already been identified.

12. The system according to any one of claims 1 and 5 to 11 wherein the sought EP landmark is at least one of the following: His bundle, right bundle, left bundle, Christa Terminalis, SA node, and AV node.

13. The system according to any one of claims 1 and 5 to 12 wherein the one or more processors are adapted to operate in real-time during an intracardiac electrogram mapping procedure having a predetermined mapping path traversing near one or more of said at least first reference spatial landmark within the heart,
optionally wherein the predetermined mapping path follows from the inferior vena cava (IVC) into the right atrium to identify the His bundle, and further follows to identify the right and left bundles and to identify the Christa Terminalis.

14. The system according to any one of claims 1 and 5 to 13 wherein said EP mapping is conducted utilizing a catheter having one or more electrodes capable of measuring the IEGM signals at a distal end thereof.

15. The system according to any one of claims 1 and 5 to 14 wherein the one or more processors are configured and operable to, upon determining that the at least one electrode is not within ROI of the sought EP landmark, provide guidance indicia for directing the catheter towards the ROI of the sought EP landmark.
